# EUROPÄISCHE PATENTSCHRIFT

(11) **EP 3 914 400 B1**
(45) Veröffentlichungstag und Bekanntmachung des Hinweises auf die Patenterteilung: **22.10.2025**
(21) Anmeldenummer: 20700290.8
(22) Anmeldetag: 13.01.2020
(51) Int. Cl.: C08F 6/00, C08F 2/16, B08B 1/00, F25B 25/00, F26B 17/04, C08F 2/00, C08F 4/40

(54) **VERFAHREN ZUR HERSTELLUNG VON SUPERABSORBERPARTIKELN**
METHOD FOR PRODUCING SUPERABSORBENT PARTICLES
PROCÉDÉ DE PRODUCTION DE PARTICULES SUPERABSORBANTES

(30) Priorität: 23.01.2019 EP 19153264
(43) Veröffentlichungstag der Anmeldung: 01.12.2021
(73) Patentinhaber: BASF SE, 67056 Ludwigshafen am Rhein (DE)
(72) Erfinder: CALLOT, Rene, 2040 Antwerpen (BE); POSSEMIERS, Karl, 2040 Antwerpen (BE); FUNK, Ruediger, 67056 Ludwigshafen (DE); KRUEGER, Marco, 67056 Ludwigshafen (DE); WEISMANTEL, Matthias, 67056 Ludwigshafen (DE); SCHROEDER, Juergen, 67056 Ludwigshafen (DE)
(74) Vertreter: BASF IP Association
(86) Internationale Anmeldenummer: PCT/EP2020/050632
(87) Internationale Veröffentlichungsnummer: WO 2020/151975

(56) Entgegenhaltungen:
- WO-A1-2015/074966
- WO-A1-2020/064411
- WO-A2-2010/139680
- DE-A1- 19 529 925
- DE-A1- 2 162 229

## Beschreibung

Die vorliegende Erfindung betrifft ein Verfahren zur Herstellung von Superabsorberpartikeln durch Polymerisation einer Monomerlösung oder -suspension, umfassend Trocknung des erhaltenen wässrigen Polymergels in einem Umluftbandtrockner, Mahlung, Klassierung, und optional thermische Oberflächennachvernetzung, wobei das wässrige Polymergel mittels eines oszillierenden Förderbandes in den Umluftbandtrockners eingebracht wird und dass sich Leiteinrichtungen an den Rändern des Förderbandes befinden.

Superabsorber werden zur Herstellung von Windeln, Tampons, Damenbinden und anderen Hygieneartikeln, aber auch als wasserzurückhaltende Mittel im landwirtschaftlichen Gartenbau verwendet. Die Superabsorber werden auch als wasserabsorbierende Polymere bezeichnet.

Die Herstellung von Superabsorbern wird in der Monographie "Modern Superabsorbent Polymer Technology", F.L. Buchholz und A.T. Graham, Wiley-VCH, 1998, Seiten 71 bis 103, beschrieben.

Zur Verbesserung der Anwendungseigenschaften, wie beispielsweise Gelbettpermeabilität (GBP) und Absorption unter einem Druck von 49,2 g/cm² (AUL0.7psi), werden Superabsorberpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der Polymerpartikel kovalente Bindungen bilden können.

WO 2008/087114 A1, WO 2010/139680 A2 und EP 2 700 667 A1 beschreiben die Beladung der Transportbänder von Umluftbandtrocknern mit wässrigem Polymergel mittels oszillierender Förderbänder.

Aufgabe der vorliegenden Erfindung war die Bereitstellung eines verbesserten Verfahrens zur Herstellung von Superabsorbern, insbesondere ein stabilerer Betrieb des verwendeten oszillierenden Förderbandes.

Gelöst wurde die Aufgabe durch ein Verfahren zur Herstellung von Superabsorbern durch Polymerisation einer Monomerlösung oder -suspension, enthaltend
a) mindestens ein ethylenisch ungesättigtes, säuregruppentragendes Monomer, das zumindest teilweise neutralisiert ist,
b) mindestens einen Vernetzer und
c) mindestens einen Initiator,
umfassend Trocknung des erhaltenen wässrigen Polymergels in einem Umluftbandtrockner, Mahlung, Klassierung, und optional thermische Oberflächennachvernetzung, dadurch gekennzeichnet, dass das wässrige Polymergel mittels eines oszillierenden Förderbandes in den Umluftbandtrockner eingebracht wird und dass sich Leiteinrichtungen (1) an den Rändern des Förderbandes (2) befinden.

Ein oszillierendes Förderband ist ein an einer vertikalen Achse periodisch schwenkbares Förderband. Das Förderband des oszillierenden Förderbandes selber läuft im wesentlichen mit konstanter Geschwindigkeit. Das Förderband des oszillierenden Förderbandes hat eine deutlich geringere Breite als das Förderband des Umluftbandtrockners. Durch die periodische Schwenkbewegung des oszillierenden Förderbandes wird das Förderband des Umluftbandtrockners über die gesamte Breite gleichmäßig mit Polymergel belegt.

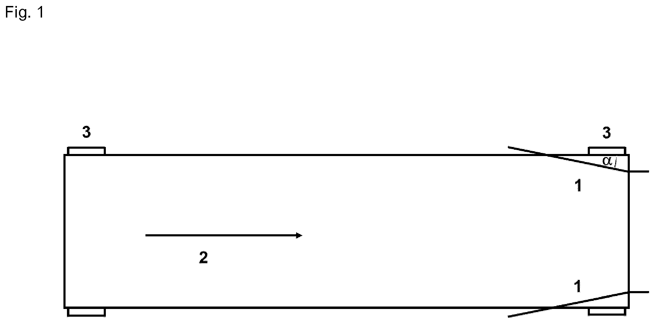

Figur 1 zeigt eine Ausführungsform der vorliegenden Erfindung mit Leiteinrichtungen (1), Förderband (2) und Antriebs- bzw. Umlenktrommel (3).

In einer bevorzugten Ausführungsform der vorliegenden Erfindung befinden sich die Leiteinrichtungen (1) am Abwurfende des Förderbandes, wobei die Länge der Leiteinrichtungen (1) vorzugsweise von 5 bis 70%, besonders bevorzugt von 10 bis 50%, ganz besonders bevorzugt von 15 bis 30% der Länge des Förderbandes beträgt, wobei die Länge des Förderbandes der Abstand der Schwenkachse vom Abwurfende ist.

Die Höhe der Leiteinrichtungen (1) beträgt vorzugsweise von 5 bis 30 cm, besonders bevorzugt von 8 bis 25 cm, ganz besonders bevorzugt von 10 bis 20 cm.

Die Länge und die Höhe der Leiteinrichtungen (1) betragen beispielsweise 5 bis 70% und 5 bis 30 cm, 5 bis 70% und 8 bis 25 cm, 5 bis 70% und 10 bis 20 cm, 10 bis 50% und 5 bis 30 cm, 10 bis 50% und 8 bis 25 cm, 10 bis 50% und 10 bis 20 cm, 15 bis 30% und 5 bis 30 cm, 15 bis 30% und 8 bis 25 cm oder 15 bis 30% und 10 bis 20 cm.

Die Leiteinrichtungen (1) können um den Winkel senkrecht zur Laufrichtung des Förderbandes zur Mitte des Förderbandes in Laufrichtung gedreht sein, wobei die Leiteinrichtungen (1) vorzugsweise um 3 bis 15°, besonders bevorzugt um 6 bis 12°, ganz besonders bevorzugt um 8 bis 10°, gedreht sind.

Der mit dem Förderband in Kontakt stehende Teil der Leiteinrichtungen (1) sollte nicht zu hart sein. Geeignete Materialien sind natürliche oder synthetische Kautschuke, wie Silikonkautschuk, Polyetheretherketon (PEEK), halogenierte Polyolefine, wie Polyvinylchlorid (PVC) oder Polytetrafluorethylen (PTFE), Polyamid (PA), Polypropylen (PP) oder Polyethylen (PE). Silikonkautschuk ist bevorzugt.

Vorzugsweise folgen die Leiteinrichtungen (1) der Krümmung des Förderbandes. Die bevorzugten Leiteinrichtungen weisen einen möglichst geringen Abstand zum Förderband auf. Ein zu großer Abstand führt dazu, dass Polymergel zwischen Leiteinrichtung (1) und Förderband nach außen gelangt. Ein zu niedriger Abstand führt zu Reibung, d.h. unerwünschter mechanischer Belastung, zwischen Leiteinrichtung (1) und Förderband.

Ist das mit dem Förderband in Kontakt stehende Material der Leiteinrichtungen (1) ausreichend flexibel, so können die Leiteinrichtungen (1) auch direkt auf dem Förderband aufliegen. Es ist sogar möglich, dass das auf dem Förderband aufliegende flexible Material der Leiteinrichtungen (1), beispielsweise Silikonkautschuk, nach innen, d.h. zum transportierten Polymergel hin, umgebogen ist.

Die Leiteinrichtungen (1) ragen vorzugsweise über das Abwurfende des Förderbandes hinaus. Die bevorzugten Leiteinrichtungen (1) folgen dabei der Krümmung an der Umlenktrommel (3) nach unten. Die Bereiche der Leiteinrichtungen (1), die über das Abwurfende des Förderbandes hinausragen, sind vorzugsweise parallel zur Laufrichtung des Förderbandes.

Der vorliegenden Erfindung liegt die Erkenntnis zugrunde, dass Polymergel unter das Förderband geraten kann. Dieses Polymergel gelangt dann auf die Trommeloberflächen der Antriebs- bzw. der Umlenktrommel. Das Polymergel auf den Trommeloberflächen führt zu Gurtschieflauf, d.h. das umlaufende Förderband wandert auf der Trommel zur Seite. Außerdem führt das Polymergel auf der Oberfläche der Antriebstrommel zum Durchrutschen der Trommel, d.h. zur Verminderung der Antriebsleistung. Die Leiteinrichtungen (1) verhindern, dass Polymergel unter das Förderband gerät. Gurtschieflauf und Durchrutschen der Antriebstrommel werden so verhindert.

In einer bevorzugten Ausführungsform der vorliegenden Erfindung wird die Unterseite des rücklaufenden Förderbandes zusätzlich mittels mindestens einer Abstreifvorrichtung von anhaftendem Polymergel gereinigt.

Die Unterseite des rücklaufenden Förderbandes ist die Außenseite des Förderbandes auf die nach Umlenkung erneut mit Polymergel dosiert wird. Die Oberseite des rücklaufenden Förderbandes ist die Innenseite des Förderbandes, die nicht mit Polymergel in Kontakt kommen soll.

Der Abstand der Abstreifvorrichtung vom Abwurfende des Förderbandes beträgt vorzugsweise weniger als 20% der Länge des Förderbandes, besonders bevorzugt weniger als 10% der Länge des Förderbandes, ganz besonders bevorzugt weniger als 5% der Länge des Förderbandes, wobei die Länge des Förderbandes der Abstand der Schwenkachse vom Abwurfende ist.

Die Abstreifvorrichtung unterliegt keinen Beschränkungen. Geeignet sind beispielsweise quer zur Laufrichtung angeordnete Bürsten. Es ist auch möglich einen Schaber zu verwenden. Ein Schaber ist eine quer zur Förderrichtung angeordnete Abstreifvorrichtung aus einem nicht-flexiblen Material. Ein geeignetes nicht-flexibles Material ist beispielsweise Polytetrafluorethylen. Um Beschädigungen des Förderbandes zu vermeiden, sollte der Schaber möglichst wenig bzw. keinen direkten Kontakt mit dem Förderband haben. Der Schaber sollte gegenüber der Laufrichtung des rücklaufenden Förderbandes geneigt sein. Dies begünstigt das Abschälen des anhaftenden Polymergels und verhindert einen Stau zwischen Förderband und Schaber. Das abgestreifte Polymergel fällt üblicherweise auf das Förderband des Umluftbandtrockners.

Der Schaber ist vorzugsweise von 5 bis 45°, besonders bevorzugt von 10 bis 35°, ganz besonders von 15 bis 25°, gegenüber der Senkrechten gegen die Laufrichtung des Förderbandes geneigt. Der Abstand des Schabers zur Unterseite des rücklaufenden Förderbandes beträgt vorzugsweise 0,1 bis 5 mm, besonders bevorzugt 0,2 bis 2 mm, ganz besonders bevorzugt 0,5 bis 1,5 mm.

Das Förderband weist eine Länge von vorzugsweise 2 bis 10 m, besonders bevorzugt von 2,5 bis 8 m, ganz besonders bevorzugt von 3 bis 6 m, auf, wobei die Länge des Förderbandes der Abstand der Schwenkachse vom Abwurfende ist.

Das Förderband weist eine Breite von vorzugsweise 0,5 bis 1,5 m, besonders bevorzugt von 0,6 bis 1,2 m, ganz besonders bevorzugt von 0,7 bis 0,9 m, auf.

Die Förderbandgeschwindigkeit beträgt vorzugsweise von 0,2 bis 2.0 m/s, besonders bevorzugt von 0,3 bis 1,5 m/s, ganz besonders bevorzugt von 0,4 bis 1.0 m/s.

Es können die für diesen Zweck üblichen Förderbänder eingesetzt werden. Die Oberfläche der Förderbänder, d.h. die mit dem Polymergel in Berührung kommende Seite, sollte wasserabweisend sein und bei 23°C einen Randwinkel gegenüber von Wasser von vorzugsweise mindestens 60°, besonders bevorzugt von mindestens 80°, ganz besonders bevorzugt von mindestens 100°, aufweisen. Der Randwinkel ist ein Maß für das Benetzungsverhalten und wird gemäß DIN 53900 gemessen.

Der Wassergehalt des Polymergels auf dem Förderband beträgt vorzugsweise von 20 bis 80 Gew.-%, besonders bevorzugt von 30 bis 70 Gew.-%, ganz besonders bevorzugt von 40 bis 60 Gew.-%.

Die Temperatur des Polymergels auf dem Förderband beträgt vorzugsweise von 60 bis 105°C, besonders bevorzugt von 70 bis 100°C, ganz besonders bevorzugt von 80 bis 95°C.

Im Folgenden wird die Herstellung der Superabsorber näher erläutert:
Die Superabsorber werden durch Polymerisation einer Monomerlösung oder -suspension hergestellt und sind üblicherweise wasserunlöslich.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere sind Acrylsäure und Methacrylsäure. Ganz besonders bevorzugt ist Acrylsäure.

Die Monomere a) enthalten üblicherweise Polymerisationsinhibitoren, vorzugsweise Hydrochinonhalbether, als Lagerstabilisator.

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomers a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 03/104299 A1, WO 03/104300 A1, WO 03/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 02/032962 A2 beschrieben.

Die Menge an Vernetzer b) beträgt vorzugsweise 0,05 bis 1,5 Gew.-%, besonders bevorzugt 0,1 bis 1 Gew.-%, ganz besonders bevorzugt 0,3 bis 0,6 Gew.-%, jeweils berechnet auf die Gesamtmenge an eingesetztem Monomer a). Mit steigendem Vernetzergehalt sinkt die Zentrifugenretentionskapazität (CRC) und die Absorption unter einem Druck von 21,0 g/cm² (AUL0.3psi) durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird vorzugsweise das Dinatriumsalz der 2-Hydroxy-2-sulfonatoessig-säure oder ein Gemisch aus dem Natriumsalz der 2-Hydroxy-2-sulfinatoessigsäure, dem Dinatriumsalz der 2-Hydroxy-2-sulfonatoessigsäure und Natriumbisulfit eingesetzt. Derartige Gemische sind als Brüggolite^{®} FF6 und Brüggolite^{®} FF7 (Brüggemann Chemicals; Heilbronn; Deutschland) erhältlich.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit der Löslichkeit überschreitendem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren für die Polymerisation sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 40 bis 85 mol-%, besonders bevorzugt von 50 bis 80 mol-%, ganz besonders bevorzugt von 60 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen. Feste Carbonate und Hydrogencarbonate können hierbei auch in verkapselter Form eingesetzt werden, vorzugsweise in die Monomerlösung direkt vor der Polymerisation, während oder nach der Polymerisation ins Polymergel und vor dessen Trocknung. Die Verkapselung erfolgt durch Beschichtung der Oberfläche mit einem unlöslichen oder nur langsam löslichen Material (beispielsweise mittels filmbildender Polymere, inerter anorganischer Materialien oder schmelzbaren organischer Materialien), welches die Lösung und Reaktion des festen Carbonats oder Hydrogencarbonats so verzögert, dass erst während der Trocknung Kohlendioxid freigesetzt wird und der entstehende Superabsorber eine hohe innere Porosität aufweist.

Das Polymergel wird dann üblicherweise mit einem Umluftbandtrockner getrocknet bis der Restfeuchtegehalt vorzugsweise 0,5 bis 10 Gew.-%, besonders bevorzugt 1 bis 7 Gew.-%, ganz besonders bevorzugt 2 bis 5 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Polymergels beträgt vor der Trocknung vorzugsweise von 25 und 90 Gew.-%, besonders bevorzugt von 35 bis 70 Gew.-%, ganz besonders bevorzugt von 40 bis 60 Gew.-%. Anschließend wird das getrocknete Polymergel gebrochen und optional grob zerkleinert.

Das getrocknete Polymergel wird hiernach üblicherweise gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise von 150 bis 850 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2 (05) "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Die Polymerpartikel können zur weiteren Verbesserung der Eigenschaften thermisch oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder β-Hydroxyalkylamide, wie in DE 102 04 938 A1 und US 6,239,230 beschrieben.

Die Menge an Oberflächennachvernetzer beträgt vorzugsweise 0,001 bis 2 Gew.-%, besonders bevorzugt 0,02 bis 1 Gew.-%, ganz besonders bevorzugt 0,05 bis 0,2 Gew.-%, jeweils bezogen auf die Polymerpartikel.

In einer bevorzugten Ausführungsform der vorliegenden Erfindung werden zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Chlorid, Bromid, Hydroxid, Sulfat, Hydrogensulfat, Carbonat, Hydrogencarbonat, Nitrat, Phosphat, Hydrogenphosphat, Dihydrogenphosphat und Carboxylat, wie Acetat und Lactat, möglich. Aluminiumhydroxid, Aluminiumsulfat und Aluminiumlaktat sind bevorzugt.

Die Einsatzmenge an polyvalentem Kation beträgt beispielsweise 0,001 bis 1,5 Gew.-%, vorzugsweise 0,005 bis 1 Gew.-%, besonders bevorzugt 0,02 bis 0,8 Gew.-%. jeweils bezogen auf das Polymer.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel oberflächennachvernetzt und getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar^{®} Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; USA) und Schugi Flexomix^{®} (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Die Oberflächennachvernetzung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex^{®} Horizontal Paddle Dryer (Hosokawa Micron GmbH; Leingarten; Deutschland), Hosokawa Bepex^{®} Disc Dryer (Hosokawa Micron GmbH; Leingarten; Deutschland), Holo-Flite^{®} dryers (Metso Minerals Industries Inc.; Danville; USA) und Nara Paddle Dryer (NARA Machinery Europe; Frechen; Deutschland). Überdies können auch Wirbelschichttrockner eingesetzt werden.

Die Oberflächennachvernetzung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und thermisch oberflächennachvernetzt.

Bevorzugte Reaktionstemperaturen liegen im Bereich 100 bis 250°C, bevorzugt 110 bis 220°C, besonders bevorzugt 120 bis 210°C, ganz besonders bevorzugt 130 bis 200°C. Die bevorzugte Verweilzeit bei dieser Temperatur beträgt vorzugsweise mindestens 10 Minuten, besonders bevorzugt mindestens 20 Minuten, ganz besonders bevorzugt mindestens 30 Minuten, und üblicherweise höchstens 60 Minuten.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%. Durch die Nachbefeuchtung wird die mechanische Stabilität der Polymerpartikel erhöht und deren Neigung zur statischen Aufladung vermindert**.** Vorteilhaft wird die Nachbefeuchtung im Kühler nach der thermischen Oberflächennachvernetzung durchgeführt.

Geeignete Beschichtungen zur Verbesserung der Quellgeschwindigkeit sowie der Gelbettpermeabilität (GBP) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil^{®} 200, Fällungskieselsäure, wie Sipernat^{®} D17, und Tenside, wie Span^{®} 20.

### Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Herrmann-Debrouxlaan 46, 1160 Oudergem, Belgien, www.edana.org) und INDA (1100 Crescent Green, Suite 115, Cary, North Carolina 27518, USA, www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

### Zentrifugenretentionskapazität (Centrifuge Retention Capacity)

Die Zentrifugenretentionskapazität (CRC) wird gemäß der von der EDANA empfohlenen Testmethode Nr. WSP 241.2 (05) "Fluid Retention Capacity in Saline, After Centrifugation" bestimmt.

### Extrahierbare

Der Gehalt an extrahierbaren Bestandteilen der wasserabsorbierenden Polymerpartikel wird gemäß der von der EDANA empfohlenen Testmethode Nr. WSP 270.2 (05) "Extractable" bestimmt.

### Beispiel

Durch kontinuierliches Mischen von entionisiertem Wasser, 50 gew.-%iger Natronlauge und Acrylsäure wurde eine Acrylsäure/Natriumacrylatlösung hergestellt, so dass der Neutralisationsgrad 71,3 mol-% entsprach. Der Feststoffgehalt der Monomerlösung betrug 38,8 Gew.-%.

Als mehrfach ethylenisch ungesättigter Vernetzer wurde Polyethylenglykol-400-diacrylat (Diacrylat ausgehend von einem Polyethylenglykol mit einem mittleren Molgewicht von 400 g/mol) verwendet. Die Einsatzmenge betrug 2 kg Vernetzer pro t Monomerlösung.

Zur Initiierung der radikalischen Polymerisation wurden pro t Monomerlösung 1,03 kg einer 0,25gew.-%igen wässriger Wasserstoffperoxidlösung, 3,10 kg einer 15gew.-%igen wässrigen Natriumperoxodisulfatlösung und 1,05 kg einer 1gew.-%igen wässrigen Ascorbinsäurelösung eingesetzt.

Der Durchsatz der Monomerlösung betrug 20 t/h. Die Reaktionslösung hatte am Zulauf eine Temperatur von 23,5°C.

Die einzelnen Komponenten wurden in folgenden Mengen kontinuierlich in einen Reaktor vom Typ List Contikneter mit einem Volumen 6,3m³ (LIST AG, Arisdorf, Schweiz) dosiert:

| | |
|---|---|
| 20 t/h | Monomerlösung |
| 40 kg/h | Polyethylenglykol-400-diacrylat |
| 82,6 kg/h | Wasserstoffperoxidlösung/Natriumperoxodisulfatlösung |
| 21 kg/h | Ascorbinsäurelösung |

Zwischen dem Zugabepunkt für den Vernetzer und den Zugabestellen für die Initiatoren wurde die Monomerlösung mit Stickstoff inertisiert.

Es fand nach ca. 50% der Verweilzeit zusätzlich eine Zudosierung von aus dem Herstellungsprozess durch Mahlung und Siebung anfallendem Feinkorn (1000 kg/h) in den Reaktor statt. Die Verweilzeit der Reaktionsmischung im Reaktor betrug 15 Minuten.

Das erhaltene wässrige Polymergel wurde mittels eines oszillierenden Förderbandes auf das Förderband eines Umluftbandtrockners aufgegeben.

Der Umluftbandtrockner hatte eine Länge von 48 m. Das Förderband des Umluftbandtrockners hatte eine effektive Breite von 4,4 m.

Das oszillierende Förderband hatte eine Länge von 5 m. Das Förderband hatte eine Breite von 0,8 m und eine effektive Breite von 0,5 m. Der Böschungswinkel des wässrigen Polymergels auf dem Förderband betrug ca. 15°. Der Querschnitt der Polymergelschüttung auf dem Förderband betrug ca. 0,04 m². Die Geschwindigkeit des Förderbandes betrug 0,5 m/s.

Das oszillierende Förderband wurde ausgehend von einer Endlage über einen ersten Schwenkwinkel ß₁ von 13° auf eine Winkelgeschwindigkeit von 33°/s beschleunigt, über einen zweiten Schwenkwinkel ß₂ von 20° auf eine Winkelgeschwindigkeit von 17°/s abgebremst und über einen dritten Schwenkwinkel ß₃ zur anderen Endlage abgebremst. Der Gesamtschwenkwinkel betrug 50°. Ein Doppelhub (von der ersten Endlage zur anderen Endlage und wieder zurück) dauerte ca. 7 s. Das umlaufende Förderband hatte eine Oberfläche aus Polytetrafluorethylen (PTFE).

Die Temperatur des wässrigen Polymergels auf dem oszillierenden Förderband betrug 90°C.

Am Abwurfende des Förderbandes befand sich an den Rändern des Förderbandes je eine Leiteinrichtung (1), wie in Figur 1 dargestellt. Die Leiteinrichtungen (1) hatten eine Länge von jeweils ca. 1.200 mm und ragten ca. 100 mm über das Abwurfende des Förderbandes hinaus. Der Teil der Leiteinrichtungen (1), der mit dem Förderband in Kontakt stand, war aus Silikonkautschuk und hatte eine Dicke von ca. 5 mm. Die Leiteinrichtungen (1) waren jeweils um ca. 8,5° senkrecht zur Laufrichtung des Förderbandes zur Mitte des Förderbandes in Laufrichtung gedreht. Der über das Abwurfende des Förderbandes hinwegreichende Teil der Leiteinrichtungen war wieder parallel zur Laufrichtung des Förderbandes. Die Leiteinrichtungen (1) folgten der Krümmung der Umlenkrolle (3) bis zur Senkrechten und waren von dort ca. 165 mm gerade nach unten verlängert.

Auf der Unterseite des oszillierenden Förderbandes befand sich eine Abstreifvorrichtung. Die Abstreifvorrichtung war ein quer zur Laufrichtung des rücklaufenden Förderbandes angebrachter länglicher Schaber. Der Schaber war um 20° gegen die Laufrichtung des rücklaufenden Förderbandes geneigt. Der Abstand der Abstreifvorrichtung vom Abwurfende betrug ca. 5 cm, d.h. die Abstreifeinrichtung befand sich im Bereich der Umlenkrolle. Der Abstand der Abstreifvorrichtung zum rücklaufenden Förderband betrug 1 mm. Mit der Abstreifvorrichtung wird an der Außenseite des rücklaufenden Förderbandes anhaftendes wässriges Polymergel abgestreift.

Das umlaufende Förderband konnte problemlos betrieben werden. Durch die Leiteinrichtungen (1) wurde verhindert, dass Polymergel auf die Oberseite des rücklaufenden Förderbandes fällt Polymergel zwischen Trommel und Förderband gelangt.

Auf dem Umluftbandtrockner wurde das wässrige Polymergel kontinuierlich mit einem Luft/Gasgemisch umströmt und getrocknet. Die Verweilzeit im Umluftbandtrockner betrug 37 Minuten.

Das getrocknete Polymergel wurde gemahlen und auf eine Partikelgrößenfraktion von 150 bis 850 µm abgesiebt.

Die erhaltenen wasserabsorbierenden Polymerpartikel wiesen eine Zentrifugenretentionskapazität (CRC) von 34,9 g/g und einen Gehalt an Extrahierbaren von 8,5 Gew.-% auf.

## Patentansprüche

1. Verfahren zur Herstellung von Superabsorberpartikeln durch Polymerisation einer Monomerlösung oder -suspension, enthaltend
a) mindestens ein ethylenisch ungesättigtes, säuregruppentragendes Monomer, das zumindest teilweise neutralisiert ist,
b) mindestens einen Vernetzer und
c) mindestens einen Initiator,
umfassend Trocknung des erhaltenen wässrigen Polymergels in einem Umluftbandtrockner, Mahlung, Klassierung, und optional thermische Oberflächennachvernetzung, **dadurch gekennzeichnet, dass** das wässrige Polymergel mittels eines oszillierenden Förderbandes in den Umluftbandtrockner eingebracht wird und dass sich Leiteinrichtungen (1) an den Rändern des Förderbandes (2) befinden, wobei ein oszillierendes Förderband ein an einer vertikalen Achse periodisch schwenkbares Förderband ist und die Leiteinrichtungen (1) verhindern, dass Polymergel unter das Förderband gerät.

2. Verfahren nach Anspruch 1, **dadurch gekennzeichnet, dass** sich die Leiteinrichtungen (1) am Abwurfende des Förderbandes befinden und die Länge der Leiteinrichtungen (1) von 15 bis 30% der Länge des Förderbandes beträgt, wobei die Länge des Förderbandes der Abstand der Schwenkachse vom Abwurfende ist, und/oder dass die Höhe der Leiteinrichtungen (1) von 10 bis 20 cm beträgt.

3. Verfahren nach Anspruch 1 oder 2, **dadurch gekennzeichnet, dass** die Leiteinrichtungen (1) um den Winkel α senkrecht zur Laufrichtung des Förderbandes zur Mitte des Förderbandes in Laufrichtung gedreht sind.

4. Verfahren nach Anspruch 3, **dadurch gekennzeichnet, dass** die Leiteinrichtungen (1) um 8 bis 10° gedreht sind.

5. Verfahren nach einem der Ansprüche 1 bis 4, **dadurch gekennzeichnet, dass** der mit dem Förderband in Kontakt stehende Teil der Leiteinrichtungen (1) aus Silikonkautschuk ist.

6. Verfahren nach einem der Ansprüche 1 bis 5, **dadurch gekennzeichnet, dass** die Leiteinrichtungen (1) der Krümmung des Förderbandes folgen.

7. Verfahren nach Anspruch 6, **dadurch gekennzeichnet, dass** die Leiteinrichtungen (1) über das Abwurfende des Förderbandes hinausragen.

8. Verfahren nach einem der Ansprüche 1 bis 7, **dadurch gekennzeichnet, dass** die Unterseite des rücklaufenden Förderbandes mittels mindestens einer Abstreifvorrichtung von anhaftendem Polymergel gereinigt wird.

9. Verfahren nach Anspruch 8, **dadurch gekennzeichnet, dass** der Abstand der Abstreifvorrichtung vom Abwurfende des Förderbandes weniger als 5% der Länge des Förderbandes beträgt, wobei die Länge des Förderbandes der Abstand der Schwenkachse vom Abwurfende ist.

10. Verfahren nach Anspruch 8 oder 9, **dadurch gekennzeichnet, dass** die auf der Unterseite des rücklaufenden Förderbandes angebrachte Abstreifeinrichtung ein Schaber ist.

11. Verfahren nach Anspruch 10, **dadurch gekennzeichnet, dass** der Schaber von 15 bis 25° gegenüber der Senkrechten gegen die Laufrichtung des Förderbandes geneigt ist.

12. Verfahren nach Anspruch 10 oder 11, **dadurch gekennzeichnet, dass** der Abstand des Schabers zur Unterseite des rücklaufenden Förderbandes von 0,1 bis 5 mm beträgt.

13. Verfahren nach einem der Ansprüche 1 bis 12, **dadurch gekennzeichnet, dass** das Förderband eine Länge von 2 bis 10 m aufweist, wobei die Länge des Förderbandes der Abstand der Schwenkachse vom Abwurfende ist.

14. Verfahren nach einem der Ansprüche 1 bis 13, **dadurch gekennzeichnet, dass** das die Oberfläche des Förderbandes bei 23°C gegenüber Wasser einen Randwinkel von mindestes 60° aufweist, wobei der Randwinkel gemäß DIN 53900 gemessen wird.

## Claims

1. A process for producing superabsorbent particles by polymerizing a monomer solution or suspension comprising
a) at least one ethylenically unsaturated monomer which bears acid groups and is at least partly neutralized,
b) at least one crosslinker and
c) at least one initiator,
comprising drying of the resultant aqueous polymer gel in an air circulation belt dryer, grinding, classifying, and optionally thermal surface postcrosslinking, wherein the aqueous polymer gel is introduced into the air circulation belt dryer by means of an oscillating conveyor belt, and wherein guide devices (1) are located at the edges of the conveyor belt (2), where an oscillating conveyor belt is a conveyor belt pivotable periodically at a vertical axis and the guide devices (1) prevent polymer gel from getting under the conveyor belt.

2. The process according to claim 1, wherein the guide devices (1) are located at the discharge end of the conveyor belt and the length of the guide devices (1) is from 15% to 30% of the length of the conveyor belt, the length of the conveyor belt being the distance of the pivot axis from the discharge end, and/or wherein the height of the guide devices (1) is from 10 to 20 cm.

3. The process according to claim 1 or 2, wherein the guide devices (1) are rotated by the angle α at right angles to the running direction of the conveyor belt to the middle of the conveyor belt in running direction.

4. The process according to claim 3, wherein the guide devices (1) are rotated by 8° to 10°.

5. The process according to any of claims 1 to 4, wherein the part of the guide devices (1) that is in contact with the conveyor belt is made of silicone rubber.

6. The process according to any of claims 1 to 5, wherein the guide devices (1) follow the curvature of the conveyor belt.

7. The process according to claim 6, wherein the guide devices (1) protrude beyond the discharge end of the conveyor belt.

8. The process according to any of claims 1 to 7, wherein the underside of the returning conveyor belt is cleaned of adhering polymer gel by means of at least one stripping device.

9. The process according to claim 8, wherein the distance of the stripping device from the discharge end of the conveyor belt is less than 5% of the length of the conveyor belt, the length of the conveyor belt being the distance of the pivot axis from the discharge end.

10. The process according to claim 8 or 9, wherein the stripper device mounted on the underside of the returning conveyor belt is a scraper.

11. The process according to claim 10, wherein the scraper is inclined at 15° to 25° relative to the horizontal counter to the running direction of the conveyor belt.

12. The process according to claim 10 or 11, wherein the distance of the scraper from the underside of the returning conveyor belt is from 0.1 to 5 mm.

13. The process according to any of claims 1 to 12, wherein the conveyor belt has a length of 2 to 10 m, the length of the conveyor belt being the distance of the pivot axis from the discharge end.

14. The process according to any of claims 1 to 13, wherein the surface of the conveyor belt at 23°C has a contact angle with respect to water of at least 60°, where the contact angle is measured in accordance with DIN 53900.

## Revendications

1. Procédé pour la fabrication de particules superabsorbantes par polymérisation d'une solution ou suspension de monomères contenant
a) au moins un monomère éthyléniquement insaturé, portant des groupes acides, qui est au moins partiellement neutralisé,
b) au moins un réticulant,
c) au moins un initiateur,
comprenant un séchage du gel polymère aqueux obtenu dans un sécheur à bande à circulation d'air, un broyage, une classification et éventuellement une postréticulation thermique en surface, **caractérisé en ce que** le gel polymère aqueux est introduit au moyen d'une bande transporteuse oscillante dans le sécheur à bande à circulation d'air et **en ce que** des dispositifs de guidage (1) sont situés au niveau des bords de la bande transporteuse (2), une bande transporteuse oscillante étant une bande transporteuse pouvant pivoter périodiquement sur un axe vertical et les dispositifs de guidage (1) empêchant que le gel polymère arrive sous la bande transporteuse.

2. Procédé selon la revendication 1, **caractérisé en ce que** les dispositifs de guidage (1) sont situés au niveau de l'extrémité d'éjection de la bande transporteuse et la longueur des dispositifs de guidage (1) représente 15 à 30% de la longueur de la bande transporteuse, la longueur de la bande transporteuse étant la distance entre l'axe de pivotement et l'extrémité d'éjection et/ou **en ce que** la hauteur des dispositifs de guidage (1) représente 10 à 20 cm.

3. Procédé selon les revendications 1 ou 2, **caractérisé en ce que** les dispositifs de guidage (1) sont tournés d'un angle α perpendiculairement à la direction de déplacement de la bande transporteuse par rapport vers le centre de la bande transporteuse dans la direction de déplacement.

4. Procédé selon la revendication 3, **caractérisé en ce que** les dispositifs de guidage (1) sont tournés de 8 à 10°.

5. Procédé selon l'une des revendications 1 à 4, **caractérisé en ce que** la partie des dispositifs de guidage (1) en contact avec la bande transporteuse est en caoutchouc de silicone.

6. Procédé selon l'une des revendications 1 à 5, **caractérisé en ce que** les dispositifs de guidage (1) suivent la courbure de la bande transporteuse.

7. Procédé selon la revendication 6, **caractérisé en ce que** les dispositifs de guidage (1) dépassent au-delà de l'extrémité d'éjection de la bande transporteuse.

8. Procédé selon l'une des revendications 1 à 7, **caractérisé en ce que** la face inférieure de la bande transporteuse qui revient en arrière est nettoyée au moyen d'un dispositif de raclage afin d'éliminer le gel polymère qui adhère.

9. Procédé selon la revendication 8, **caractérisé en ce que** la distance entre le dispositif de raclage et l'extrémité d'éjection de la bande transporteuse représente moins de 5% de la longueur de la bande transporteuse, la longueur de la bande transporteuse étant la distance entre l'axe de pivotement et l'extrémité d'éjection.

10. Procédé selon la revendication 8 ou 9, **caractérisé en ce que** le dispositif de raclage disposé sur la face inférieure de la bande transporteuse qui revient en arrière est un grattoir.

11. Procédé selon la revendication 10, **caractérisé en ce que** le grattoir est incliné de 15 à 25° par rapport à la perpendiculaire à la direction de déplacement de la bande transporteuse.

12. Procédé selon la revendication 10 ou 11, **caractérisé en ce que** la distance entre le grattoir et la face inférieure de la bande transporteuse qui revient en arrière est de 0,1 à 5 mm.

13. Procédé selon l'une des revendications 1 à 12, **caractérisé en ce que** la bande transporteuse présente une longueur de 2 à 10 m, la longueur de la bande transporteuse étant la distance entre l'axe de pivotement et l'extrémité d'éjection.

14. Procédé selon l'une des revendications 1 à 13, **caractérisé en ce que** la surface de la bande transporteuse présente, à 23°C et par rapport à l'eau, un angle de mouillage d'au moins 60°, l'angle de mouillage étant mesuré selon la norme DIN 53900.
